# EUROPEAN PATENT SPECIFICATION

(11) **EP 3 199 186 B1**
(45) Date of publication and mention of the grant of the patent: **31.07.2019**
(21) Application number: 17153647.7
(22) Date of filing: 27.01.2017
(51) Int. Cl.: A61L 31/04, A61L 31/06, A61L 31/16, C08L 33/08, A61K 9/00, A61K 45/06, A61K 31/085, A61K 31/275, A61K 31/4995, A61K 31/78, C09D 133/06, C08L 33/06, C08L 33/14, A61L 31/14

(54) **INJECTABLE COMPOSITIONS AND METHODS OF USE THEREOF**
EINSPRITZBARE ZUSAMMENSETZUNGEN UND VERFAHREN ZUR VERWENDUNG DAVON
COMPOSITIONS INJECTABLES ET LEURS PROCÉDÉS D'UTILISATION

(30) Priority: 28.01.2016 US 201662287950 P
(43) Date of publication of application: 02.08.2017
(73) Proprietor: Cook Medical Technologies LLC, Bloomington, IN 47404 (US)
(72) Inventor: TANNER, Shaun A., West Lafayette, IN Indiana 47906 (US); ISCH, Andrew P., West Lafayette, IN Indiana 47906 (US); NEVAREZ, Erik, Hammond, IN Indiana 46327 (US); LEUNG, Gary, Lafayette, IN Indiana 47905 (US)
(74) Representative: Simpson, Tobias Rutger

(56) References cited:
- WO-A1-01/68720
- US-A1- 2008 050 436

## Description

### FIELD OF THE INVENTION

The present disclosure relates generally to injectable compositions and to methods of using such compositions for thermal ablation of tissue and for the occlusion of vascular and other vessels.

### BACKGROUND

One approach to removing an undesired source of tissue growth involves the application of sufficient thermal energy to the target growth to remove the tissue by ablation. Various types of growths that can be removed through thermal ablation include tumors, diseased muscle tissue and warts. The technique may also be used to treat damaged tissue affecting cardiac rhythms and blood pressure. WO 01/68720 relates to embolic compositions comprising crosslinkable macromers, which form hydrogels at the target site of the vessel. The macromer backbone might include hydroxyethyl methacrylate. The crosslinkable functional group might be a methacrylate. The compositions can be used in combination with a subsequent thermal ablation.

In the ablation technique, the tissue is eliminated by necrosis, and allowed to slough away. Compared with surgical removal of tissue, ablation therapy may have reduced morbidity, lower cost, and may spare surrounding tissue. Ablation has been used to remove a variety of tumor types such as in liver, lung, breast, pancreas, bile duct, bone, and kidney.

Existing ablation techniques include the use of Radio frequency (RF) energy. RF ablation techniques, however, suffer from the disadvantage that special precautions are needed to use an RF generator in conjunction with a magnetic resonance imager because of the risk from the displacement force of the magnet. Cryo-ablation has also been used to ablate tissue but is susceptible to a high rate of reoccurrence. Techniques have also been described for ablating tissue with the heat generated from a chemical reaction. Existing thermochemical ablation techniques, however, can suffer from the complications associated with handling of multiple potentially hazardous chemicals in liquid or gaseous form and the removal of the reaction by-products from the treatment site without contamination of the target tissue from the reactants or their by-products.

### SUMMARY

One aspect of the present invention provides a combination of compositions as defined in Claim 1, which is useful for chemical ablation and occlusion. The combination may include two components that may be combined to form an injectable composition that may be administered, for example, by a subcutaneous, an intramuscular, an intradermal, an intravascular or an intravenous route.

In one embodiment, the combination is a two- composition combination, the first composition including 2-hydroxyethyl methacrylate, a dimethacrylate crosslinker, 1,4-diazabicyclooctane, and 1-[4-(2-Hydroxyethoxy)-phenyl]-2-hydroxy-2-methyl-1-propane-1-one, and the second composition including a diisocyanate, a triisocyanate or a poly-isocyanate.

In one embodiment, the dimethacrylate crosslinker is a bioabsorbable polymer dimethacrylate, for example PLGA-PEG-PLGA dimethacrylate. In another embodiment, the dimethacrylate crosslinker is ethylene glycol dimethacrylate. The first composition may also include a small molecular weight diol, triol or polyol compound.

A bioactive agent may be included with the first or the second composition. In certain embodiments, the bioactive is an anti-cancer agent, an antithrombogenic agent, an antimicrobial agent, an antibiotic, an antiproliferative agent, an antiinflammatory agent, or an immunosuppressive agent or a combination of at least two of these materials.

Another aspect of the invention provides the combination for use in a method of therapy, as defined in Claim 9. Preferably, the method comprises combining the first composition and the second composition to form an injectable composition and administering a therapeutically effective amount of the injectable composition to a patient. In some embodiments, the method of therapy is a method of destroying tissue within the body of a patient, preferably wherein the method comprises combining the first composition and the second composition to form an injectable composition and administering a therapeutically effective amount of the injectable composition to the patient. In other embodiments, the method of therapy is a method of occluding a vascular vessel, preferably a vein, within the body of a patient, preferably wherein the method comprises combining the first composition and the second composition to form an injectable composition and administering the injectable composition into the vessel.

Another aspect of the invention provides an injectable composition suitable for use in a method of therapy, wherein the injectable composition is formed by combining the first composition and the second composition, as defined in Claim 12. In some embodiments, the method of therapy is a method of destroying tissue within the body of a patient, wherein the method comprises administering a therapeutically effective amount of the injectable composition to the patient. In other embodiments, the method of therapy is a method of occluding a vascular vessel, preferably a vein, within the body of a patient, wherein the method comprises administering the injectable composition into the vessel.

The present disclosure also provides a method of destroying tissue within the body of a patient. In one embodiment, the method includes combining the composition disclosed herein to form an injectable composition and administering a therapeutically effective amount of the injectable composition to the patient. The administration may be by, for example, a subcutaneous, an intramuscular, an intradermal, an intravascular or an intravenous route. In some embodiments, the composition is administered within 1 minute of combining the two compositions to form the injectable composition.

The combination of the two compositions may result in an exothermic reaction that raises the temperature of the surrounding tissue to at least 50 deg. C or at least 60 deg. C. The injectable composition may be delivered directly into the tissue of a cancerous tumor or a benign tumor. The injectable composition may also be administered to vascular vessels supplying the tumor and may form a solid mass within the vascular vessels resulting in at least a reduction of the blood supply to the tumor.

Another aspect of the present disclosure provides a method of occluding a vascular vessel within the body of a patient. In one embodiment, the method includes combining the compositions disclosed herein to form an injectable composition and administering a therapeutically effective amount of the injectable composition to the vascular vessel. The vascular vessel may be, for example, a vein.

In some embodiments, the composition is administered within 1 minute of combining the two compositions to form the injectable composition. In other embodiments the injectable composition forms a solid mass within the vessel within seven minutes of combining the compositions.

### BRIEF DESCRIPTION OF THE DRAWINGS

The invention may be more fully understood by reading the following description in conjunction with the drawings, in which:

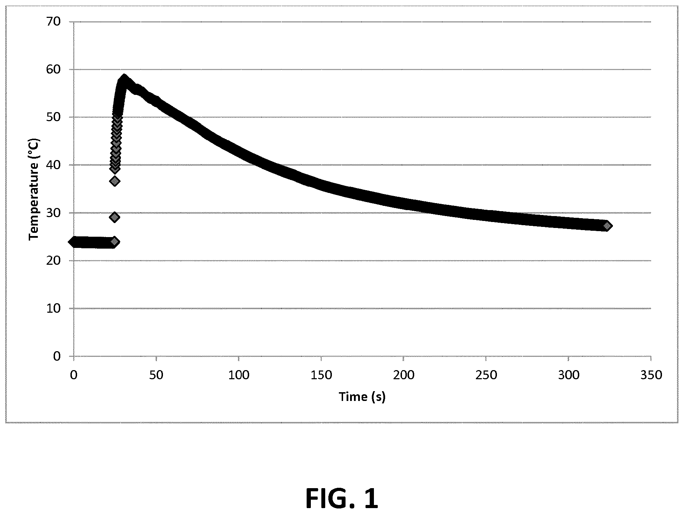

Figure 1 is a graph showing the temperature within a pocket formed in porcine liver tissue reaches a temperature of approximately 60 deg. C after injection with an injectable composition of one embodiment of the present invention.

### DEFINITIONS

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention. The materials, methods, and examples disclosed herein are illustrative only and not intended to be limiting.

As used herein the terms "comprise(s)," "include(s)," "having," "has," "can," "contain(s)," and variants thereof, are intended to be open-ended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

As used herein, the term "bioactive" refers to any pharmaceutically active agent that produces an intended therapeutic effect on the body to treat or prevent conditions or diseases.

As used herein, the term "therapeutically effective amount", refers to an amount which, when administered to a patient alleviates disease or condition by thermochemical ablation of tissue or by at least partially occluding a vessel within the body of the patient.

As used herein, the term patient refers to a human or veterinary patient.

### DETAILED DESCRIPTION

For the purposes of promoting an understanding of the principles of the invention, reference will now be made to the embodiments illustrated in the drawings and specific language will be used to describe the same. It will nevertheless be understood that no limitation of the scope of the invention is thereby intended, and alterations and modifications in the illustrated device, and further applications of the principles of the invention as illustrated therein are herein contemplated as would normally occur to one skilled in the art to which the invention relates.

### Combinations and Compositions for Occlusive and Ablative Treatment

One aspect of the present invention provides a combination of agents useful for chemical ablation and occlusion. In some embodiments, the components of the combination are mixed to form a composition that is administered to a human or veterinary patient for the occlusive and ablative treatment of conditions, such as malignant or benign tumors and other dysfunctional tissue disorders. The composition may be an injectable composition and may, in some embodiments, be administered by a subcutaneous, an intramuscular, an intradermal, an intravascular or an intravenous route.

The injectable composition may be formed by mixing a first composition and a second composition. Upon combining the two compositions an exothermic reaction occurs. Administration of the injectable composition to the tissue of a patient will result in an increase in the temperature of tissue in the vicinity of the site of the injectable composition, resulting in the destruction of the tissue.

Combining the two compositions may also result in the transition of the combined composition from a liquid to, firstly, a gel, and eventually to solid mass. In some embodiments, this composition is injected into the vasculature supporting a tumor, resulting in a reduction or elimination in the blood supply to the tumor.

Thus, the injectable (combined) composition may offer two different mechanisms for treatment. Raising the localized temperature in a region containing dysfunctional tissue assists in the destruction of such tissue. In addition, placing the injectable composition in the vasculature supplying blood to the dysfunctional tissue results in at least a reduction in the blood supply to the tissue, also assisting in the destruction of the tissue.

In one aspect of the present invention, the composition is formed by mixing two compositions. In one embodiment, the first composition includes 2-hydroxyethyl methacrylate ("HEMA"), a dimethacrylate crosslinker, 1,4-diazabicyclooctane, and 1-[4-(2-Hydroxyethoxy)-phenyl]-2-hydroxy-2-methyl-1-propane-1-one. In this embodiment the second composition includes an isocyanate, which may be, for example, a diisocyanate, a triisocyanate or a poly-isocyanate.

One, or both, of the two compositions may include at least one pharmaceutically acceptable carrier, diluent or excipient. In addition, one, or both, of the compositions may include at least one bioactive. Examples of such bioactives include, but are not limited to, an anti-cancer agent, an antithrombogenic agent, an antimicrobial agent, an antibiotic, an antiproliferative agent, an antiinflammatory agent, and an immunosuppressive agent. A combination of at least two of these components may be present.

The dimethacrylate crosslinker may be a biostable or a bioabsorbable polymer dimethacrylate. For example, the dimethacrylate crosslinker may be a biostable polymer dimethacrylate, such as, but not limited to, ethylene glycol dimethacrylate.

In other embodiments, the dimethacrylate crosslinker may be a bioabsorbable polymer dimethacrylate, including, but not limited to, Poly(ε-caprolactone) dimethacrylate, Polylactide dimethacrylate, Poly(lactide-co-glycolide) dimethacrylate, Poly(ε-caprolactone-b-ethylene glycol-b-ε-caprolactone) dimethacrylate, Poly(lactide-b-ethylene glycol-b-lactide) dimethacrylate, Poly[(lactide-co-glycolide)-b-ethylene glycol-b-(lactide-co-glycolide)] dimethacrylate, Poly(ε-caprolactone-co-lactide)-dimethacrylate, Poly(ε-caprolactone-co-glycolide)-dimethacrylate, Poly[(caprolactone-co-lactide)-b-ethylene glycol-b-(caprolactone-co-lactide)] dimethacrylate, Poly[(caprolactone-co-glycolide)-b-ethylene glycol-b-(caprolactone-co-glycolide)] dimethacrylate.

In one embodiment, the first composition includes 2-hydroxyethyl methacrylate, dimethacrylate crosslinker, 1,4-diazabicyclooctane and1-[4-(2-Hydroxyethoxy)-phenyl]-2-hydroxy-2-methyl-1-propane-1-one in the weight ratio of 950 to 995 parts 2-hydroxyethyl methacrylate, to 5 to 10 parts dimethacrylate crosslinker, to 1 to 3 parts 1,4-diazabicyclooctane, to 0.5 to 2 parts 1-[4-(2-Hydroxyethoxy)-phenyl]-2-hydroxy-2-methyl-1-propane-1-one.

In another embodiment, the first composition includes, for example, between 90 and 99.5%, or between 95 to 99.5%, or between 97 and 99%, or between 98 and 99% v/v 2-hydroxyethyl methacrylate. The first composition may also include between 0.5 and 1%, or between 0.6 and 0.9%, or between 0.7 and 0.9% v/v dimethacrylate crosslinker. The amount of 1,4-diazabicyclooctane may be between, for example, 0.05 to 0.4%, or 0.1 to 0.3%, or 0.1 to 0.3% wt./wt. The amount of 1-[4-(2-Hydroxyethoxy)-phenyl]-2-hydroxy-2-methyl-1-propane-1-one may be between, for example, 0.025 and 0.15%, or 0.075 and 0.12%, or 0.05% to 0.2% wt./wt.

In some embodiments, the percentages of the dimethacrylate crosslinker and 1-[4-(2-Hydroxyethoxy)-phenyl]-2-hydroxy-2-methyl-1-propane-1-one are varied to affect the injectability of the material. In other embodiments, the percentage of diazabicyclooctane is varied to affect the rate of curing after the material is injected.

In other embodiments, the first composition includes 2-hydroxyethyl methacrylate, dimethacrylate crosslinker, 1,4-diazabicyclooctane, and 1-[4-(2-Hydroxyethoxy)-phenyl]-2-hydroxy-2-methyl-1-propane-1-one in the ratios disclosed above and also include a small molecular weight diol, triol or polyol compound and/or the pharmaceutically acceptable carrier, diluent or excipient and at least one of the bioactives disclosed herein. For example, polyethylene glycol can be included as a linear (diol) or multi-armed polymer or oligomer. A combination of polylactide, glycolide, or caprolactone can be included as a linear, 3-armed, or multi-armed polymer or oligomer. In some embodiments, the small molecular weight diol, triol or polyol compound is present at between 1 to 20, or 1 to 10, or 5 to 10 percentage by weight of the composition.

In one preferred embodiment, the first composition includes approximately 98.9% (v/v) 2-Hydroxyethyl Methacrylate (CAS# 868-77-9), 0.75% Ethylene Glycol Dimethacrylate (EGDMA) (CAS# 97-90-5), 0.25% DABCO®33-LV(CAS#280-57-9) and 0.1% IRGACURE® 2959 (BASF, CAS# 106797-53-9, 1-[4-(2-Hydroxyethoxy)-phenyl]-2-hydroxy-2-methyl-1-propane-1-one). DABCO® is a mixture of 33% 1,4-Diazabicyclooctane (CAS# 280-57-9) diluted with propylene glycol (CAS# 57-55-6). In this embodiment, the second composition includes lysine triisocyanate.

The following example illustrates one embodiment of a method of the preparation and use of this composition. A similar method of preparation and use is applicable to the compositions disclosed herein. A solution containing 3 mL HEMA, 11.5 microL EGDM, and 25.8 microL IRGACURE® 2959 solution (I g/mL in DMSO) is passed through a basic alumina column to remove any reaction inhibitors. 500 microL of this mixture is then added to 46.5 microL DABCO® 33-LV to form the first component. This solution is exposed to ultraviolet radiation (365 nm, 200 W) for 30 sec, resulting in a partially cross-linked, viscous liquid. The second component includes 630 microL of lysine triisocyanate (CAS# 69878-18-8, 85% purity)

The two compositions are placed in separate syringes and then mixed. Typically, the compositions are mixed for between 2 sec and 15 seconds before delivery of the injectable composition to the target area. However, the mixing time may vary depending on the formulation of the compositions and the application for which they are used. In other embodiments, the two compositions are mixed for between 1 second and 1 minute, or between 10 seconds and 45 seconds, or between 15 seconds and 30 seconds before delivery.

After delivery, the combined composition undergoes a curing process to form a gel and eventually a solid mass. The curing time, i.e. the time taken to form a solid mass, may depend upon the formulation of the composition. Typically, curing is complete in between 5 minutes and 10 minutes after mixing, for example within 5, 6, 7, 8, 9 or 10 minutes after mixing. However, the curing time may vary beyond this range for some compositions. In other embodiments, the curing time is, for example, between 2 minutes and 20 minutes, 5 minutes and 15 minutes, or 10 minutes and 20 minutes. The curing process involves an exothermic reaction, resulting in an increase in the temperature of the composition and the surrounding tissue. In some embodiments, the temperature within the composition reaches 65 deg. C, or 60 deg. C, or 55 deg. C, or 50 deg. C.

The present disclosure also provides a method of treating a disease or condition in a patient including destroying tissue within the body of the patient. In one embodiment, the method includes administering a therapeutically effective amount of an injectable composition as disclosed herein to the patient. As is disclosed herein, the composition may be an injectable composition formed by mixing a first and a second composition as disclosed herein. The injectable composition may then be administered to the patient.

The method may be used to destroy damaged or dysfunctional tissue within the body of the patient by raising the temperature within the tissue, resulting in the destruction of cells within the tissue. The tissue may include a cancerous or benign tumor, including, but not limited to, a tumor within the liver, kidney, breast, pancreas, bile duct, lung or bone.

In certain embodiments, the injectable composition may be administered directly into the tumor or other tissue requiring treatment. Such administration will result in an increase in the temperature within the vicinity of the administration such that the damaged cells within the tissue are destroyed. In other embodiments, the injectable composition is administered to vascular vessels supplying the tissue. In these embodiments, the composition forms a solid mass within the vascular vessels, at least limiting the blood supply to the tissue. Such a reduction in blood supply limits the supply of necessary nutriments resulting in the death of cells within the tissue. In some embodiments, the therapeutic action of the composition may be as a result of a combination of the heat generating and vascular occlusive properties.

Another aspect the present disclosure provides a method of at least partially occluding a vascular or other vessel and at least limiting fluid flow through the vessel. In a preferred embodiment, the vessel is totally occluded resulting in the elimination of fluid flow.

In one embodiment, the method includes combining the compositions disclosed herein to form an injectable composition and administering a therapeutically effective amount of the injectable composition to the vessel. The vessel may be a vascular vessel may be, for example, a vein. In some embodiments, the method may be used to occlude a blood vessel having a weakened vessel wall or aneurysm. The vessel may be, for example, a cerebral vessel, a renal vessel, or an aortic or a peripheral vascular vessel.

Yet another aspect of the present disclosure provides a kit including at least two reagents, provided in separate compartments. The first reagent may include the first composition of the combination as disclosed herein. For example, the first reagent may include 2-hydroxyethyl methacrylate; a dimethacrylate crosslinker; 1,4-diazabicyclooctane; and 1-[4-(2-Hydroxyethoxy)-phenyl]-2-hydroxy-2-methyl-1-propane-1-one. The second reagent may include the second composition of the combination as disclosed herein. For example, the second reagent may include a diisocyanate, a triisocyanate or a poly-isocyanate.

In certain embodiments, the kit may also include suitable devices for mixing the two reagents to form a combined reagent and/or administering the combined reagent to a patient. For example, the kit may also include a syringe, catheter, needle, or IV delivery system including a stopcock for delivery of the combined reagent to a patient. In certain embodiments, the syringe may include compartments containing the reagents and a mixing chamber for mixing the components prior to administration.

Embodiments of the invention will be further described in the following examples, which do not limit the scope of the invention described in the claims.

### Example 1 - Demonstration of ablative action in porcine liver

An ex-vivo porcine liver tissue model is used to determine the ablative potential of the combination. An injectable composition including a first and second component is prepared as disclosed herein. The first component includes approximately 98.9% (v/v) 2-Hydroxyethyl Methacrylate(HEMA) (CAS# 868-77-9), 0.75% Ethylene Glycol Dimethacrylate (EGDMA) (CAS# 97-90-5), 0.25% DABCO®33-LV(CAS#280-57-9) and 0.1% IRGACURE® 2959 (BASF, CAS# 106797-53-9, 1-[4-(2-Hydroxyethoxy)-phenyl]-2-hydroxy-2-methyl-1-propane-1-one). DABCO® is prepared by mixing of 33% 1,4-Diazabicyclooctane (CAS# 280-57-9) diluted with propylene glycol (CAS# 57-55-6). The second component includes lysine triisocyanate (CAS# 69878-18-8, 85% purity).

A solution containing 3 mL HEMA, 11.5 microL EGDM, and 25.8 microL IRGACURE® 2959 solution (I g/mL in DMSO) is passed through a basic alumina column to remove any reaction inhibitors. 500 microL of this mixture is then added to 46.5 microL DABCO® 33-LV to form the first component. This solution is exposed to ultraviolet radiation (365 nm, 200 W) for 30 sec, resulting in a partially cross-linked, viscous liquid. The second component includes 630 microL of lysine triisocyanate.

A scalpel is used to create a small pocket in the porcine liver tissue and a temperature probe inserted into the pocket. The two components are placed in separate syringes connected to a common chamber and mixed for approximately 15 seconds before injection into the pocket in the liver tissue.

The temperature within the pocket is measured using the temperature probe. Figure 1 shows that the temperature within the pocket reaches approximately 60 deg. C. Parts of the pocket are discolored indicating ablative tissue damage. The composition is examined after the curing process is complete and removed from the pocket as a solid mass.

## Claims

1. A combination comprising:
a first composition comprising:
2-hydroxyethyl methacrylate;
a dimethacrylate crosslinker;
1,4-diazabicyclooctane; and
1-[4-(2-Hydroxyethoxy)-phenyl]-2-hydroxy-2-methyl-1-propane-1-one, and a second composition comprising
an isocyanate selected from the group consisting of a diisocyanate, a triisocyanate and a poly-isocyanate,
wherein the first composition and the second composition are arranged separately.

2. The combination of claim 1, wherein the dimethacrylate crosslinker is a bioabsorbable polymer dimethacrylate, preferably wherein the bioabsorbable polymer dimethacrylate is PLGA-PEG-PLGA dimethacrylate.

3. The combination of claim 1, wherein the dimethacrylate crosslinker is ethylene glycol dimethacrylate.

4. The combination of any preceding claim, wherein the first composition further comprises a bioactive, preferably wherein the bioactive is selected from the group consisting of an anti-cancer agent, an antithrombogenic agent, an antimicrobial agent, an antibiotic, an antiproliferative agent, an antiinflammatory agent, and an immunosuppressive agent.

5. The combination of any preceding claim, wherein the first composition comprises 2-hydroxyethyl methacrylate, dimethacrylate crosslinker, 1,4-diazabicyclooctane and 1-[4-(2-Hydroxyethoxy)-phenyl]-2-hydroxy-2-methyl-1-propane-1-one in the weight ratio of:
950 to 995 parts 2-hydroxyethyl methacrylate; to
5 to 10 parts dimethacrylate crosslinker; to
1 to 3 parts 1,4-diazabicyclooctane; to
0.5 to 2 parts 1-[4-(2-Hydroxyethoxy)-phenyl]-2-hydroxy-2-methyl-1-propane-1-one, and
wherein the isocyanate is lysine triisocyanate.

6. The combination of any preceding claim, the first composition further comprising a small molecular weight compound selected from the group consisting of a diol, a triol and a polyol, preferably wherein the small molecular weight compound is present at between 1 to 20 percentage by weight.

7. The combination of any preceding claim, wherein the combination is a two-component combination consisting of the first composition and the second composition.

8. The combination of any preceding claim, wherein mixing of the first composition and the second composition forms an injectable composition, preferably wherein the injectable composition is administrable by a route selected from the group consisting of a subcutaneous, an intramuscular, an intradermal, an intravascular and an intravenous route.

9. A combination of any of claims 1-8 for use in a method of therapy, preferably wherein the method comprises combining the first composition and the second composition to form an injectable composition and administering a therapeutically effective amount of the injectable composition to a patient.

10. The combination for use of claim 9, wherein the method of therapy is a method of destroying tissue within the body of a patient.

11. The combination for use of claim 9, wherein the method of therapy is a method of occluding a vascular vessel, preferably a vein, within the body of a patient, preferably wherein the method comprises combining the first composition and the second composition to form an injectable composition and administering the injectable composition into the vessel.

12. An injectable composition suitable for use in a method of therapy,
wherein the injectable composition is formed by combining a first composition and a second composition,
wherein the first composition comprises
2-hydroxyethyl methacrylate;
a dimethacrylate crosslinker;
1,4-diazabicyclooctane; and
1-[4-(2-Hydroxyethoxy)-phenyl]-2-hydroxy-2-methyl-1-propane-1-one, and
the second composition comprises an isocyanate selected from the group consisting of a diisocyanate, a triisocyanate and a poly-isocyanate.

## Patentansprüche

1. Kombination, umfassend:
eine erste Zusammensetzung, umfassend:
2-Hydroxyethylmethacrylat;
ein Dimethacrylat-Vernetzungsmittel;
1,4-Diazabicyclooctan; und
1-[4-(2-Hydroxyethoxy)phenyl]-2-hydroxy-2-methyl-1-propan-1-on, und
eine zweite Zusammensetzung, umfassend
ein Isocyanat, ausgewählt aus der Gruppe bestehend aus einem Diisocyanat, einem Triisocyanat und einem Polyisocyanat,
wobei die erste Zusammensetzung und die zweite Zusammensetzung getrennt angeordnet sind.

2. Kombination nach Anspruch 1, wobei das Dimethacrylat-Vernetzungsmittel ein bioabsorbierbares Polymerdimethacrylat ist, wobei das bioabsorbierbare Polymerdimethacrylat vorzugsweise PLGA-PEG-PLGA-Dimethacrylat ist.

3. Kombination nach Anspruch 1, wobei das Dimethacrylat-Vernetzungsmittel Ethylenglycoldimethacrylat ist.

4. Kombination nach einem vorhergehenden Anspruch, wobei die erste Zusammensetzung zudem ein Bioaktivum umfasst, wobei das Bioaktivum vorzugsweise ausgewählt ist aus der Gruppe bestehend aus einem Antikrebsmittel, einem Antithrombosemittel, einem Antimikrobiotikum, einem Antibiotikum, einem Antiproliferationsmittel, einem Entzündungshemmer und einem Immunsuppressivum.

5. Kombination nach einem vorhergehenden Anspruch, wobei die erste Zusammensetzung 2-Hydroxyethylmethacrylat, Dimethacrylat-Vernetzungsmittel, 1,4-Diazabicyclooctan und 1-[4-(2-Hydroxyethoxy)phenyl]-2-hydroxy-2-methyl-1-propan-1-on im Gewichtsverhältnis von:
950 bis 995 Teilen 2-Hydroxyethylmethacrylat; zu
5 bis 10 Teilen Dimethacrylat-Vernetzungsmittel; zu
1 bis 3 Teilen 1,4-Diazabicyclooctan; zu
0,5 bis 2 Teilen 1-[4-(2-Hydroxyethoxy)phenyl]-2-hydroxy-2-methyl-1-propan-1-on umfasst, und
wobei das Isocyanat Lysintriisocyanat ist.

6. Kombination nach einem vorhergehenden Anspruch, wobei die erste Zusammensetzung zudem eine Verbindung mit niedrigem Molekulargewicht umfasst, ausgewählt aus der Gruppe bestehend aus einem Diol, einem Triol und einem Polyol, wobei die Verbindung mit niedrigem Molekulargewicht vorzugsweise zwischen 1 und 20 Gewichtsprozent vorliegt.

7. Kombination nach einem vorhergehenden Anspruch, wobei die Kombination eine Zweikomponentenkombination ist, die aus der ersten Zusammensetzung und der zweiten Zusammensetzung besteht.

8. Kombination nach einem vorhergehenden Anspruch, wobei das Mischen der ersten Zusammensetzung und der zweiten Zusammensetzung eine injizierbare Zusammensetzung bildet, wobei sich die injizierbare Zusammensetzung vorzugsweise auf einem Weg verabreichen lässt, der ausgewählt ist aus der Gruppe bestehend aus einem subkutanen, einem intramuskulären, einem intradermalen, einem intravaskulären und einem intravenösen Weg.

9. Kombination nach einem der Ansprüche 1 bis 8 zur Verwendung bei einem Therapieverfahren, wobei das Verfahren vorzugsweise das Kombinieren der ersten Zusammensetzung und der zweiten Zusammensetzung zur Bildung einer injizierbaren Zusammensetzung und das Verabreichen einer therapeutisch wirksamen Menge der injizierbaren Zusammensetzung an einen Patienten umfasst.

10. Kombination zur Verwendung nach Anspruch 9, wobei das Therapieverfahren ein Verfahren zum Zerstören von Gewebe im Körper eines Patienten ist.

11. Kombination zur Verwendung nach Anspruch 9, wobei das Therapieverfahren ein Verfahren zum Verschließen eines vaskulären Gefäßes, vorzugsweise einer Vene, im Körper eines Patienten ist, wobei das Verfahren vorzugsweise das Kombinieren der ersten Zusammensetzung und der zweiten Zusammensetzung, um eine injizierbare Zusammensetzung zu bilden, und das Verabreichen der injizierbaren Zusammensetzung in das Gefäß umfasst.

12. Injizierbare Zusammensetzung, die sich zur Verwendung bei einem Therapieverfahren eignet,
wobei die injizierbare Zusammensetzung durch Kombinieren einer ersten Zusammensetzung und einer zweiten Zusammensetzung gebildet wird,
wobei die erste Zusammensetzung
2-Hydroxyethylmethacrylat;
ein Dimethacrylat-Vernetzungsmittel;
1,4-Diazabicyclooctan; und
1-[4-(2-Hydroxyethoxy)phenyl]-2-hydroxy-2-methyl-1-propan-1-on umfasst, und
die zweite Zusammensetzung ein Isocyanat, ausgewählt aus der Gruppe bestehend aus einem Diisocyanat, einem Triisocyanat und einem Polyisocyanat umfasst.

## Revendications

1. Combinaison comprenant :
une première composition comprenant :
du méthacrylate de 2-hydroxyéthyle ;
un agent de réticulation de type diméthacrylate ;
du 1,4-diazabicyclooctane ; et
de la 1-[4-(2-hydroxyéthoxy)-phényl]-2-hydroxy-2-méthyl-1-propane-1-one, et une seconde composition comprenant
un isocyanate choisi dans le groupe constitué par un diisocyanate, un triisocyanate et un polyisocyanate,
où la première composition et la deuxième composition sont disposées séparément.

2. Combinaison selon la revendication 1, où l'agent de réticulation de type diméthacrylate est un diméthacrylate polymère bioabsorbable, préférentiellement où le diméthacrylate de polymère bioabsorbable est le diméthacrylate de PLGA-PEG-PLGA.

3. Combinaison selon la revendication 1, où l'agent de réticulation de type diméthacrylate est le diméthacrylate d'éthylène glycol.

4. Combinaison selon l'une quelconque des revendications précédentes, où la première composition comprend en outre un agent bioactif, préférentiellement où l'agent bioactif est choisi dans le groupe constitué par un agent anticancéreux, un agent antithrombogène, un agent antimicrobien, un antibiotique, un agent antiprolifératif, un agent anti-inflammatoire et un agent immunosuppresseur.

5. Combinaison selon l'une quelconque des revendications précédentes, où la première composition comprend du méthacrylate de 2-hydroxyéthyle, un agent de réticulation de type diméthacrylate, du 1,4-diazabicyclooctane et du 1-[4-(2-hydroxyéthoxy)-phényl]-2-hydroxy-2-méthyl-1-propane-1-one dans un rapport massique de :
950 à 995 parts de méthacrylate de 2-hydroxyéthyle ; sur
5 à 10 parts d'agent de réticulation de type diméthacrylate ; sur 1 à 3 parts de 1,4-diazabicyclooctane ; sur
0,5 à 2 parts de 1-[4-(2-hydroxyéthoxy)-phényl]-2-hydroxy-2-méthyl-1-propane-1-one, et
où l'isocyanate est le triisocyanate de lysine.

6. Combinaison selon l'une quelconque des revendications précédentes, la première composition comprenant en outre un composé de faible masse moléculaire choisi dans le groupe constitué par un diol, un triol et un polyol, préférentiellement où le composé de faible masse moléculaire est présent à entre environ 1 et 20 pour cent en masse.

7. Combinaison selon l'une quelconque des revendications précédentes, où la combinaison est une combinaison bicomposant constituée de la première composition et de la deuxième composition.

8. Combinaison selon l'une quelconque des revendications précédentes, où le mélangeage de la première composition et de la deuxième composition forme une composition pour injection, préférentiellement où la composition pour injection peut être administrée par une voie choisie dans le groupe constitué par une voie sous-cutanée, intramusculaire, intradermique, intravasculaire et intraveineuse.

9. Combinaison selon l'une quelconque des revendications 1 à 8 pour utilisation dans une méthode de traitement thérapeutique, préférentiellement où la méthode comprend la combinaison de la première composition et de la deuxième composition pour former une composition pour injection et l'administration d'une quantité thérapeutiquement active de la composition pour injection à un patient.

10. Combinaison pour utilisation selon la revendication 9, où la méthode de traitement thérapeutique est une méthode de destruction des tissus à l'intérieur du corps d'un patient.

11. Combinaison pour utilisation selon la revendication 9, où la méthode de traitement thérapeutique est une méthode d'occlusion d'un vaisseau, préférentiellement d'une veine, à l'intérieur du corps d'un patient, préférentiellement où la méthode comprend la combinaison de la première composition et de la deuxième composition pour former une composition pour injection et l'administration de la composition pour injection dans le vaisseau.

12. Composition pour injection adaptée à une utilisation dans une méthode de traitement thérapeutique,
où la composition pour injection est formée par combinaison d'une première composition et d'une deuxième composition,
où la première composition comprend
du méthacrylate de 2-hydroxyéthyle ;
un agent de réticulation de type diméthacrylate ;
du 1,4-diazabicyclooctane ; et
de la 1-[4-(2-hydroxyéthoxy)-phényl]-2-hydroxy-2-méthyl-1-propane-1-one, et
la deuxième composition comprend un isocyanate choisi dans le groupe constitué par un diisocyanate, un triisocyanate et un polyisocyanate.
